# EUROPEAN PATENT APPLICATION

(11) **EP 2 437 061 A1**
(43) Date of publication of application: **04.04.2012**
(21) Application number: 10012370.2
(22) Date of filing: 30.09.2010
(51) Int. Cl.: G01N 33/569, G01N 33/58, C07K 14/35

(54) **Mycobacterial protein detection**

(71) Applicant: Ikonomopoulos, John, 152 34 Halandri (GR)
(72) Inventor: Sechi, Leonardo, Antonio, I-07100 Sassari (IT); Fabio, Cimaglia, I-7202 Torchiarolo (BR) (IT); Chiesa, Maurizio, I-73100 Lecce (IT)
(74) Representative: Malamis, Alkisti-Irene

(57) **Abstract**

The invention provides use of a technique for detecting mycobacterial proteins using quantum dot (QD) labels. This allows rapid and direct detection of the main mycobacterial pathogens, for example *Mycobacterium tuberculosis* complex (MTC), *M. avium* complex (MAV) and *M. avium* subsp *paratuberculosis* (MAP), in clinical samples, in a way that is highly specific, very easy to perform, and requires minimum infrastructure and expertise.

## Description

### Field of the invention

The general technical field of the invention relates to mycobacterium protein detection.

### Background to the invention

Pathogens of the genus *Mycobacterium* are a major cause of morbidity and mortality in humans and animals worldwide. Tuberculosis results nowadays to millions of deaths or disabilities each year, especially in poorer areas of the planet according to WHO reports, and this more than 100 years after the first description by Koch of the bacteria responsible. The problem is exacerbated by the AIDS epidemic that increases disease incidence in developed countries too. (www. who.org/tb/en).

Concerning humans, in addition to tuberculosis, exposure to mycobacteria has also been linked to the pathogenesis of sarcoidosis and Crohn's disease that affect millions of people in Europe only. The disease threat posed by the pathogens of the genus *Mycobacterium* is not unique to humans. Non-human primates can be infected by *Mycohacterium tuberculosis,* while cattle and other ruminants are natural hosts of the closely-related species *M. bovis,* which causes substantial financial loss in many regions of the world. The *M avium* complex affects birds and mammals, including both livestock and dogs. Finally, paratuberculosis is another chronic infectious disease caused by a member of *Mycobacterium* spp., namely *M*. *avium* subsp *paratuberculosis* (MAP) that affects mainly ruminants.

### Summary of the invention

The invention provides use of a technique for detecting mycobacterial proteins using quantum dot (QD) labels. This allows rapid and direct detection of the main mycobacterial pathogens *[Mycobacterium tuberculosis* complex (MTC), *M. avium* complex (MAV) and *M. avium* subsp *paratuberculosis* (MAP)] in clinical samples, in a way that is highly specific, very easy to perform, and requires minimum infrastructure and expertise.

Accordingly the invention provides a method of determining the presence of mycobacterial proteins (therefore mycobacterial species) in a sample comprising:
(i) using a capture antibody, which is a mycobacterial monoclonal antibody.
   A sample is added, and any antigen present binds to capture antibody. The antigen hound by capture antibody is then detected with a biotin conjugated second antibody, which is a mycobacterial monoclonal antibody, different from the one used as capture antibody , followed by addition of streptavidin linked Quantum Dot;
   or
(ii) using a capture antibody which is a mycobacterial monoclonal antibody. A sample is added, and any antigen present binds to capture antibody.
   Any antigen bound by capture antibody is detected with a second antibody which ia a mycobacterial monoclonal antibody, different from the one used for coating, followed by an anti-mouse IgG biotin conjugated antibody. Detection is performed by addition of streptavidin linked Quantum Dot;
   or
(iii) detecting antigen in the sample, for example sera, adsorbed in a solid surface. If present, the antigen is detected by a biotin conjugated monoclonal antibody followed by streptavidin linked Quantum Dot detection or by a monoclonal antibody which is detected with a second antibody biotin conjugated followed by streptavidin linked Quantum Dot detection
and wherein optionally the determination of whether the sample comprises a mycobaterial protein is carried out by detection of a change in photoluminescence.

Moreover the invention provides a method of determining the presence of specific antibodies against mycobacteria antigens, by using specific mycobacterial proteins (antigens). A sample is added and any antibody present against the specific mycobacterial antigen will bind to it.

The complex antibody- antigen will be detected by using a biotin conjugated secondary antibody IgG, for example anti human IgG for human sera, followed by the streptavidin linked quantum dot, to allow determination of the complex second antibody - first antibody. Advantageously, between each step rounds of washes are performed to remove antibodies not specifically bound.

### Detailed description of the invention

The invention allows detection of proteins from a mycobacterium, for example from *Mycobacteria spp, Mycobacterium tuberculosis comples* (MTB), *M*. *avium complex* (MAC) *or M avium subsp paratuberculosis* (MAP).

QDs are semiconductor nanocrystals whose photoluminescence emission wavelength is proportional to the size of the crystal. The emission spectra of the QDs are narrow which allows multiwavelength labeling and effectively multi-target labeling with different sizes of QDs with little overlap, Their outer surface can be readily conjugated to organic molecules, resulting in a spectrum of biologically compatible labels that are excited with a single wavelength (Bruchez et al., 1998, Chan and Nie, 1998).

QDs are already integrated in research and routine diagnostic applications. (Parungo et al., 2005). Targeting and imaging of cancerous cells has been used by several research groups both in vivo (Gao et al., 2004, Stroh et al., 2005) and in vitro (Tokumasu et al., 2005). Selective distraction of these cells has also been exploited and offers a fascinating prospect for treatment (Sonvico ct al., 2005). Recently, QDs have been used for the detection of several different bacterial species providing also information on their viability (Kloepfer et al., 2003, Su and Li, 2004, Dwarakanath et al., 2004).

QDs arc highly sensitive biosensors, since they already constitute the basis of a very innovative diagnostic approach. The ability to detect very small amounts of the QDs can decrease dramatically the lower detection limit of the diagnostic assays in which they are incorporated, allowing direct, even in-field detection of microbial nucleotide sequences, without the need for amplification. QDs are very promising as they could be used for the simultaneous quantitative detection of different targets. A great number of samples can be processed at the same time while the use of portable devices could permit the in-field detection of the QDs.

In one embodiment of the method it is carried out to diagnose infection of an individual suspected to have a mycobacterial infection, wherein the individual is preferably human. The invention provides a method of determining the presence of mycobacterial proteins in a sample comprising using a capture antibody, which is a mycobacterial monoclonal antibody.

In one embodiment, this determination is carried out by adding a sample, and any antigen present binds to capture antibody, wherein the antigen bound by capture antibody is then detected with a biotin conjugated second antibody, which is a mycobacterial monoclonal antibody, different from the one used as capture antibody, followed by addition of streptavidin linked Quantum Dot;

In mother embodiment, this determination is carried out by adding a sample, and any antigen present binds to capture antibody, wherein any antigen bound by capture antibody is detected with a second antibody which ia a mycobacterial monoclonal antibody, different from the one used for coating, followed by an anti-mouse IgG biotin conjugated antibody and wherein detection is performed by addition of streptavidin linked Quantum Dot;

In another embodiment antigen is detected in the sample, for example sera, adsorbed in a solid surface and a capture antibody is used to bind antigen in the sample, followed by the use of biotin conjugated monoclonal antibody (specific for the antigen) and by the streptavidin linked Quantum Dot, or the antigen is detected by a monoclonal antibody which is detected with a second antibody biotin conjugated followed by streptavidin linked Quantum Dot detection

In the embodiments described above detection of whether the first antibody has bound to a protein in the sample will be done by measuring the presence of the quantum dot (which is now effectively bound to the protein via one or two antibodies). This may be achieved by detection of a change in photoluminescence, for example using UV light detection or using a fluorescence plate detector.

The invention may be carried out to diagnose whether or not an individual has mycobacterial infection, in which cases the sample will be from the relevant individual. The individual is typically a mammal, such as a human. The individual may or may not have symptoms of a mycobacterial disease. The sample generally comprises purified protein. It may be in the form of a lysate.

In one embodiment of the method the sample is bound to a solid surface, such as paper, a gel, a plastic surface or a microtitre plate.

The first antibody may specifically bind to glycosyl transferase d, heparin-binding hemagglutinin adhesin, methylated heparin-binding hemagglutinin adhcsin or ferric reductase. The first antibody may specifically bind a protein of M. avium subspecies paratuberculosis or M. tuberculosis, and the protein of M. avium subspecies paratuberculosis is optionally glycosyl transferase d or heparin-binding hemagglutinin adhesion and the protein of M. tuberculosis is optionally methylated heparin-binding hemagglutinin adhesin or ferric reductase.

The first and/or second antibodies are typically monoclonal antibodies. An antibody used in the method of the invention may either be a whole antibody or a fragment thereof which is capable of binding the same epitope. Typically the antibody is a mammalian antibody, such as a primate, human, rodent (e.g. mouse or rat), rabbit, ovine, porcine, equine or camel antibody. The antibody can be any class or isotype of antibody, for example IgM, but is preferably IgG.

The fragment of whole antibody that can be used in the method comprises an antigen binding site, e.g. Fab or F(ab)₂ fragments. The whole antibody or fragment may be associated with other moieties, such as linkers which may be used to join together 2 or more fragments or antibodies. Such linkers may be chemical linkers or can be present in the form of a fusion protein with the fragment or whole antibody. The linkers may thus be used to join together whole antibodies or fragments which have the same or different binding specificities.

In the method 2, 3, 4 or more different first antibodies are used, optionally labeled with different quantum dots labels. Typically the different quantum dots each have a different emission spectrum.

The invention also provides a kit for carrying out the method comprising 1, 2 or 3 different first antibodies or second antibodies as defined herein, and optionally also instructions for carrying out the method. The kit may also comprise a means to detect quantum dots.

Any of the following antibodies (which are produced by their respective hybridomas) may be used as capture antibody and as first antibody:
*M.tuberculosis* HBHA
   1B11.1
   4A8
   1G10
   4C10
   3H4
   5F3
*M. tuberculosis* FDR
   4E8
   2B12
   3A10
   3A10
   5G10
   4H9
   4H9
   SB8
   2B12
   1A7
   2G4
*M.paratuberculosis* HBHA
   4H12
   5E10
   SB6
   3D6
   3D6
   1D4
   5B4
*M.paratuberculosis* Gsd
   3G12

Typically, a QD is a semiconductor nanocrystal, whose radii are smaller than the bulk exciton Bohr radius, which constitutes a class of materials intermediate between molecular and bulk forms of matter. QDs are typically formed from inorganic, crystalline semiconductive materials and have unique photophysical, photochemical, and nonlincar optical properties arising from quantum size effects.

A QD may exhibit a number of unique optical properties due to quantum confinement effects. For example, QDs possess strong luminescence, photostability against bleaching and physical environments such as pH and temperature, and optical tunability, overcoming many of the shortcomings apparent with organic fluorophores.

QD8 may be formed from an inner core of one or more first semiconductor materials that optionally may be contained within an overcoating or "shell" of a second semiconductor material. A QD core surrounded by a semiconductor shell is referred to as a "core/shell" QD. The optional surrounding shell material will preferably have a bandgap energy that is larger than the bandgap energy of the core material and may be chosen to have an atomic spacing chose to that of the core substrate. Suitable semiconductor materials for the core and/or the optional shell include, but are not limited to, the following: materials comprised of a first element selected from Groups 2 and 12 of the Periodic Table of the Elements and a second element selected from Group 16 (e.g., ZnS, ZnSe, ZnTe, CDs, CdSe, CdTe, HgS, HgSc, HgTe, MgS, MgSe, MgTe, CaS, CaSe, CaTe, SrS, SrSc, SrTe, BaS, BaSe, BaTe, and the like); materials comprised of a first element selected from Group 13 of the Periodic Table of the Elements and a second element selected from Group 15 (e.g., GaN, Gap, GaAs, GaSh, lnN, InP, InAs, InSb, and the like); materials comprised of a Group 14 element (Ge, Si, and the like); materials such as PbS, PbSe and the like; and alloys and mixtures thereof. As used herein, all reference to the Periodic Table of the Elements and groups thereof is to the IUPAC system for numbering element groups, as sct forth in the Handbook of Chemistry and Physics, 81 st Edition (CRC Press, 2000). QDs may he made using techniques known in the art. See, e.g., U.S. Pat. Nos. 6,048,616; 5,990,479; 5,690,807; 5,505,928; and 5,262,357. QDs used in the present disclosure may absorb a wide spectrum of light, and may be physically tuned with emission bandwidths in various wavelengths. See, e.g., Badolato, et al., Science 208:1158-61 (2005). For example, the emission bandwidth may be in the visible spectrum (e.g., from about 3.5 to 7.5 [mu]m), the visible-infrared spectrum (e.g., from about 0.1 to about 0.7 [mu]m), or in the near-infrared spectrum (e.g., from about 0.7 to about 2.5 [mu]m). QDs that emit energy in the visible range include, but are not limited to, CdS, CdSe, CdTe, ZnSc, ZnTe, GaP, and GaAs. QDs that emit energy in the blue to near-ultraviolet range include, but are not limited to, ZnS and GaN. QDs that emit energy in the near-infrared range include, but arc not limited to, InP, InAs, InSb, PbS, and PbSc. QDs with emission spectra in the near- infrared spectrum may be particularly suited for probes used in certain imaging applications. For example, such QDs may be suited for in vivo imaging, among other things, due to tissue's high optical transmissivity in the near-infrared spectrum. For attachment, the QD may comprise a functional group or attachment moiety. One example of such a QD that has a functional group or attachment moiety is a QD with a carboxylic acid terminated surface, such as those commercially available though, for example, Quantum Dot, Inc., Hayward, CA. The QD is preferably a CdSe QD.

The presence of the QD will generally be carried out by a detector capable of detecting luminescence from a QD. To detect luminescence, the detector should be positioned in relation to the sample such that luminescence can be detected, and the detector should be adapted to detect luminescence. One example of a suitable detector is a fluorimeter. In some embodiments, the system may further comprise a source of electromagnetic radiation, for example, an excitation monochromator. An excitation monochromator may be used to excide the probe at a specific wavelength to ensure the quality of emission detection.

Specific mycobacterial proteins are mentioned herein. In one embodiment the first antibody specifically binds homologues of any of these specific proteins. Such homologues typically have at least 70% homology, preferably at least 80, 90%, 95%, 97% or 99% homology, for example over a region of at least 15, 20, 30 or more contiguous amino acids, or even across the entire length of the original sequence. The homology may he calculated on the basis of amino acid identity (sometimes referred to as "hard homology").

For example the UWGCG Package provides the BESTFIT program which can be used to calculate homology (for example used on its default settings) (Devereux et al (1984) Nucleic Acids Research 12, p387-395). The PILEUP and BLAST algorithms can be used to calculate homology or line up sequences (such as identifying equivalent or corresponding sequences (typically on their default settings), for example as described in Altschul S. F. (1993) J Mol Evol 36:290-300; Altschul, S, F et al (1990) J Mol Biol 215;403-10.

Software for performing BLAST analyses is publicly available through the National Center for Biotechnology Information (This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al*, supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands. The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The homologous sequence typically differs by 1, 2, less than 5, less than 10 or less than 15 mutations (each of which may be a substitution, deletion or insertion of a nucleotide), These mutations may be measured across any of the regions mentioned above in relation to calculating homology.

### PROTEIN SEQUENCES

### MAP-HBHA (MAP-3968) SEQ ID NO: 1

MKGTTMAENPNIDDLRAPLLAALGAADLALATVNDLIANLRERAEETRAETRTRVEERRARLTKFQ
EDLPEQFTELRDKFTTEELRKAAEGYLEAATNRYNELVERGEAALQRLRSQTAFEDASARAEGYVD
QAVELTQEALGTVASQTRAVGERAAKLVGIELPGKAEAAGKKAQKAIAKAPAKKAPAKKVTQK

### MAP-GSD (MAP-1234) SEQ ID NO: 2

MTAPVFSIIIPTFNAAVTLQACLGSIVGQTYREVEVVLV[?]GGSTDRTLDIANSFRPELCSRLVVHSGPDD
GPYDAMNRGVGVATGEWVLFLGADDTLYEPTTLAQVAAFIGDHAASHLVYGDVVMRSTKSRHAGPFDLDR
LLFETNLCHQSIFYRRELFDGIGPYNLRYRVWADWDFNIRCFSNPALITRYMDVVISEYNDMTGFSMRQG
TDKEFRKRLPMYFWVAGWETCRRMLAFLKDKENRRLALRTRLIRVKAVSKERSAEP

FDR-MTB, NADPH:adrenodoxin oxidoreductase FPRA (NADPH-ferredoxin reductase) [Mycobacterium tuberculosis H37Rv1: SEQ ID NO: 3

mrpyyiaivg sgpsaffaaa sllkaadtte dldmavdmle mlptpwglvr sgvapdhpki
ksiskqfekt aedprfrffg nvvvgehvqp gelserydav iyavgaqsdr mlnipgedlp
gsiaavdfvg wynahphfeq vspdlsgara vvigngnval dvarilltdp dvlartdiad
haleslrprg iqevvivgrr gplqaafttl elreladldg vdvvidpael dgitdedaaa
vgkvckqnik vlrgyadrep rpghrrmvfr fltspieikg krkverivlg rnelvsdgsg
rvaakdtger eelpaqlvvr svgyrgvptp glpfddqsgt ipnvggring spneyvvgwi
krgptgvigt nkkdaqdtvd tliknlgnak egaecksfpe dhadqvadwl aarqpklvts
ahwqvidafe raagephgrp rvklaslael lriglg

All publications which are mentioned above and below are incorporated herein by reference.

The following Examples illustrate the invention.

### Example 1

Here we report the development of a microplate-based fluorescence immunoassay using a QD-streptavidin approach that is used to show the binding of anti-mycobacterial monoclonal antibodies to mycobacterial antigens in biological samples. QDs with a streptavidin bridge, immobilized on their surface via electrostatic interactions, were 9 purchased from Invitrogen. Polyclonal biotinylated antibodies used to interact with streptavidin-QDs were purchased from Sigma. Microplate-based fluorescence assay could be performed by using two different types of immunoplates, depending on the method chosen for fluorescence detection, as described below:
1. Nunc MaxiSorp™ high protein-binding capacity polystyrene 96 well ELISA plates, for qualitative detection under UV light.
2. Corning® 96-Well Black Solid high binding Plates, for quantitative detection by a fluorescence plate reader (such as the Tecan instrument).

### MTB and MAP monoclonal antibodies

Proteins of *M.tuberculosis* (MTB) and *Mycobacterium avium* subs. *paratuberculosis* (MAP) used for monoclonal antibodies production were:

### For MTB:

**HBHA :** heparin-binding hemagglutinin adhesin, a ∼30 kDa recombinant antigen required for extra pulmonary dissemination.

MTB-specific ferric reductase

### for MAP:

**HBHA:** heparin-binding hemagglutinin adhesin, a ∼30 kDa recombinant antigen required for extra pulmonary dissemination (cloned in our lab)

**GSD** :glycosyl transferase d, a 72 kDa recombinant antigen which has amino-acid sequence homology with human gastrointestinal glutathione peroxidase protein (cloned in our lab).

All mycobacterial antigens, except MAP-GSD, were constructed as 6xhistidine (6xHis)-tagged recombinant proteins. MAP-GSD was fused to a maltose-binding tag (MBP) by cloning the MAP *gsd* gene into the pMAL-c2 expression vector (New England Biolabs, Beverly, MA, USA). Each recombinant protein was over expressed by using 1 mM IPTG (for 6xHis-tagged antigens) or 0.3 mM IPTG (for MAP-GSD-MBP antigen) and purified by either nickel chromatography or by maltose affinity chromatography, following standard protocols.

### Monoclonal detected antibodies were:

### For MTB:

Methylated HBHA (6 monoclona) antibodies)

MTB-specific ferric reductase (9 monoclonal antibodies)

### For MAP:

HBHA (6 monoclonal antibodies, one IgM)

GSD (1 monoclonal antibody)

### Identification of Mycobacterial proteins:

### QDs ELISA Protocol (by using a secondary antibody biotin conjugated)

1. Plates are coated overnight at 4°C with approximately 10µg/ml of either purified mycobacterials protein or mycobacterial lysate diluted in carbonate bicarbonate buffer (Sigma- Aldlrich)
2. The next day the solution is removed and plates are blocked with 200 µl of 5% non-fat milk in PBS for 1 h at room temperate (RT)
3. After blocking, plates are washed twice with PBS-T (plus 0.05% Tween-20)
4. 1,4 µl (from 1mg/ml stock solution) of monoclonal antibody is diluted in 100ul of PBS-T and added to each well, plates are then incubated at RT for 2 hours. (NB. two negative controls were included in each late: 1. no monoclonal antibody, 2, no protein or lysate.)
5. Plates are washed 4 times in PBS-T
6. 100µl (1:2000 dilution) of polyclonal anti mouse IgG biotinylated antibody is added to each well, plates are then incubated for 1 hour at RT.
7. Plates are washed 5 times in PBS-T
8. 100µl of streptavidin QD conjugate (1:300 dilution in sterile H₂O) is added to each well, plates are incubated for at least 1 hour at RT or overnight at 4°C.
9. After washing, plates are prepared for fluorescence detection as follows:
   - for Nunc plates: dried plates can be directly visualized under UV light. Positive samples show the appropriate fluorescence colour or QDs, whereas in negative samples no fluorescence can be detected.
   - for dark Corning plates: 10µl of H₂O is added to wells for fluorescence reading. Measurement of QDs fluorescence is performed in a fluorescent plate reader (such as Tecan instrument).
      Excitation: the broad absorption spectra of QDs allow a wide choice of excitation lines
      Emission: filter choice depends on the emission spectra of the QD used in the assay

All plates could be stored at 4°C.

### Identification of Mycobacterial proteins:

### QDs ELTSA Protocol (by using monoclonal antibodies biotin conjugated) Protocol should be as follows:

1. Plates are coaled overnight at 4°C with approximately 10µg/ml of either purified mycobacterial protein or mycobacterial lysate diluted in carbonate bicarbonate buffer (Sigma-Aldrich)
2. The next day the solution is removed and plates are blocked with 200µl of 5% non-fat milk in PBS for 1 h at room temperate (RT)
3. After blocking, plates are washed twice with PBS-T (plus 0.05% Tween-20)
4. 1,4 ul (from 1mg/ml stock solution) of monoclonal antibody (biotin conjugated) is diluted in 100ul of PRS-T and added to each well, plates are then incubated at RT for 2 hours. (NB two negative controls were included in each late: 1. no monoclonal antibody, 2. no protein or lysate.)
5. Plates are washed 4 times in PBS-T
6. 100µl of streptavidin QD conjugate (1:300 dilution in sterile H₂O) is added to each well, plates are incubated for at least 1 hour at RT or overnight at 4°C.
7. After washing, plates are prepared for fluorescence detection as follows:
8. for Nunc plates: dried plates can be directly visualized under UV light. Positive samples show the appropriate fluorescence colour of QDs, whereas in negative samples no fluorescence can be detected.
9. for dark Coming plates: 10µl of H₂O is added to wells for fluorescence reading. Measurements of QDs fluorescence is performed in a fluorescent plate reader (such as Tecan instrument).
10. Excitation: the broad absorption spectra of QDs allow a wide choice of excitation lines
11. Emission: filter choice depends on the emission spectra of the QD used in the assay

All plates could be stored at 4°C.

### Representative results:

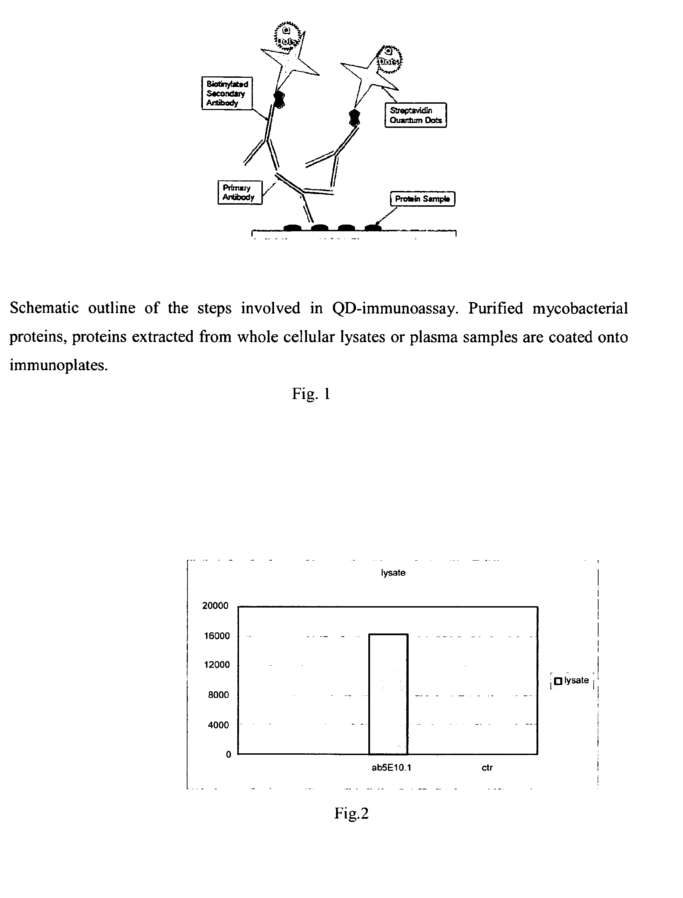

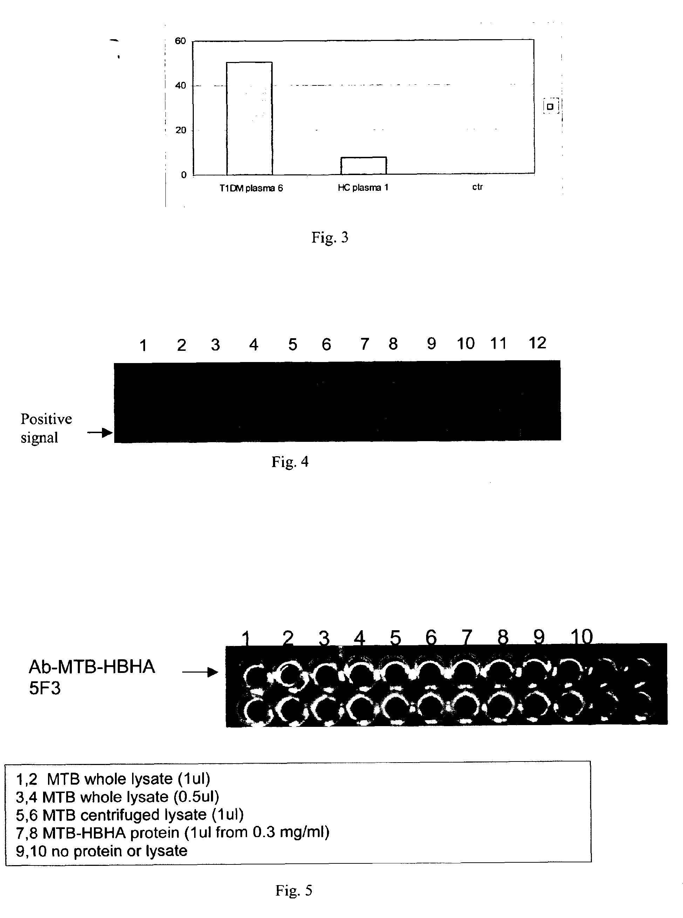

1. Whole Map lysate was coated in a dark Coming plate. Monoclonal antibody 5E10 (anti-MAP-HBHA) was used as primary antibody. Fluorescence detection was performed by a Tecan instrument (gain optimal). Background fluorescence value from negative control was subtracted from the fluorescence value of the biological sample. See Fig 2.
2. Plasma sample from a T1DM patient and from a non diabetic control (HC) were coated in a dark Coming plate. Monoclonal antibody 5E10 (anti-MAP-HBHA) was used as primary antibody. Fluorescence detection was performed by a Tecan instrument (gain optimal). Background fluorescence value from negative control (CTR) was subtracted from the fluorescence value of plasma samples. See Fig. 3.
3. MAP-HBHA was coated in a Nunc maxisorp plate. Monoclonal antibody 5E10 (anti-MAP-HBHA) was used as primary antibody. Fluorescence detection was visualized under UV light. Row 2: lines 1-8 positive samples; lines 9-12 negative controls. See Fig. 4.

### Sandwich ELISA using QDs

1. Plates are coated overnight at 4°C with approximately 20µg/ml of monoclonal antibody diluted in carbonate bicarbonate buffer (Sigma-Aldrich)
2. The next day the solution is removed and plates are blocked with 200µl of 5% non-fat milk in PBS for 1 h at room temperate (RT)
3. After blocking, plates are washed twice with PBS-T(plus 0.05% Tween-20)
4. 1ul of sample (recombinant protein, mycobacterial lysate, clinical sample, etc) is diluted in 100ul of PBS-T and added to each well, plates are then incubated at RT for 2 hours. (NB. a negative control is included in each plate: no sample)
5. Plates are washed 4 times in PHS-T
6. 100µl (10ug/ml)) of biotinylated monoclonal antibody (different from the one used for coating) is added to each well, plates are then incubated for 1-2 hours at RT.
7. Plates are washed 4 times in PBS-T
8. 100µl of streptavidin QD conjugate (1:400 dilution in steril H₂O) is added to each well, plates are incubated for at least 1 hour at RT or over night at 4 °C.
9. After washing, plates are prepared for fluorescence detection as follow:
   - for Nunc plates : plates can be directly visualized under UV light. Positive samples show the appropriate fluorescence color of QDs, whereas in negative samples no fluorescence can be detected.
   - for dark Coming plates : 100µl of H₂O is added to wells. Measurement of QDs fluorescence is performed in a fluorescent plate reader (such as Tecan instrument).

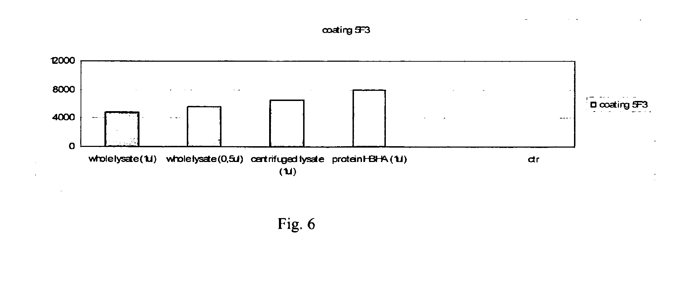

### Representative results (see figs 5 and 6):

1. abMTB-HBHA 5F3 (capture antibody) Was used to coat Nunc and dark Coming plate wells. Biotinylated abMTB-HBHA monoclonal antibody 1B11 was used as primary antibody. Fluorescence detection was performed by UV light (qualitative detection) and by a Tecan instrument (quantitative detection). Fluorescence emission value from negative control was subtracted from the fluorescence value of the biological sample (background correction).

### Literature

Bruchez, M. Jr., Moronne, M., Gin, P., Weiss, S., Alivisatos, A.P., 1998. Semiconductor nanocrystals as fluorescent biological labels. Science. 281, 2013-2016.
Chan, W.C., Nie, S., 1998. Quantum dot bioconjugates for ultrasensitive nonisotopic detection. Science. 281, 2016-2018.
Cole, S.T., Brosch, R., Parkhill, J., Garnier, T., Churcher, C., IIarris, D., Gordon, S.V., Eiglmeier, K., Gas, S., Barry, C.I., Tckaia, F., Badcock, K., Basham, D., Brown, D., Chillingworth, T., Connor, R., Davies, R., Devlin, K., Feltwell, T., Gentles, S., Hamlin, N., Holroyd, S., Hornsby, T., Jagels, K., Krogh, A., McLean, J., Moule, S., Murphy, L., Oliver, K., Osborne. J., Quail, M.A., Rajandream, M.A., Rogers, J., Rutter, S., Seeger, K., Skelton, J., Squares, R., Squares, S., Sulston, J.E., Taylor, K., Whitehead, S., Barrell, B.C., 1998. Deciphering the biology of Mycobacterium tuberculosis from the complete genome sequence. Nat. 393, 537-544.
Dwarakanath, S., Bruno, J.G., Shastry, A., Phillips, T., John, A.A., Kumar, A., Stephenson, L.D., 2005. Quantum dot-antibody and aptamer conjugates shift fluorescence upon binding bacteria. Biochem. Biophys. Res. Commun. 325, 739-743.
Gao, X., Cui, Y., Levenson, R.M., Chung, L.W., Nie, S., 2004. In vivo cancer targeting and imaging with semiconductor quantum dots. Nat. Biotechnol. 22, 969-976.
Kloepfer, J.A., Mielke, R.E., Wong, M.S., Nealson, K.H., Stucky, G., Nadeau, J.L., 2003. Quantum dots as strain- and metabolism-specific microbiological labels. Appl. Environ. Microbiol, 69, 4205-4213.
Parungo, C.P., Ohnishi, S., Kim, S.W., Kim, S., Laurence, R.G., Soltcsz, E.G., Chen, F.Y., Colson, Y.L., Cohn, L.II., Bawendi, M.G., Frangioni, J.V., 2005. Intraoperative identification of esophageal sentinel lymph nodes with near-infrared fluorescence imaging. J. Thorac. Cardiovasc. Surg. 129, 844-850.
Sonvico, F., Dubemet, C., Colombo, P., Couvreur, P., 2005. Metallic colloid nanotechnology, applications in diagnosis and therapeutics. Curr. Pharm. Des. 11, 2095-2105.
Stroh, M., Zimmer, J.P., Duda, D.G., Levchenko, T.S., Cohen, K.S., Brown, E.B., Scadden, D.T., Torchilin, V.P., Bawendi, M.G., Fukumura, D., Jain, R.K., 2005. Quantum dots spectrally distinguish multiple species within the tumor milieu in vivo. Nat. Med. 11, 678-682.
Su, X.L., Li, Y., 2004. Quantum dot biolabcling coupled with immunomagnetic separation for detection of Escherichia coli O157:H7. Anal. Chem. 76, 4806-4810.
Tokumasu, F., Fairhurst, R.M., Ostera, G.R., Brittain, N.J., Hwang, J., Wellems, T.E., Dvorak, J.A., 2005. Band 3 modifications in Plasmodium falciparum-infected AA and CC crythrocytes assayed by autocorrelation analysis using quantum dots. J. Cell. Sci. 118, 1091-1098.

## Claims

1. A method of determining the presence of mycobacterial proteins in a sample comprising
(i) using a capture antibody, which is a mycobacterial monoclonal antibody.
A sample is added, and any antigen present hinds to capture antibody. The antigen bound by capture antibody is then detected with a biotin conjugated second antibody, which is a mycobacterial monoclonal antibody, different from the one used as capture antibody , followed by addition of streptavidin linked Quantum Dot;
or
(ii) using a capture antibody which is a mycobacterial monoclonal antibody. A sample is added, and any antigen present binds to capture antibody.
Any antigen bound by capture antibody is detected with a second antibody which ia a mycobacterial monoclonal antibody, different from the one used for coating, followed by an anti-mouse IgG biotin conjugated antibody. Detection is performed by addition of streptavidin linked Quantum Dot;
or
(iii) detecting antigen in the sample, for example sera, adsorbed in a solid surface. If present, the antigen is detected by a biotin conjugated monoclonal antibody followed by streptavidin linked Quantum Dot Detection or by a monoclonal antibody which is detected with a second antibody biotin conjugated followed by streptavidin linked Quantum Dot detection
and wherein optionally the determination of whether the sample comprises a mycobatetial protein is carried out by detection of a change in photoluminescence.

2. A method according to claim 1 which is carried out to diagnose infection of an individual suspected to have a mycobacterial infection, wherein the individual is preferably human,

3. A method according to claim 1 which is performed using an ELISA format.

4. A method according to any one of claims 1 to 3 wherein said sample has been coated to a solid surface, optionally to microtitre plate.

5. A method according to any one of the preceding claims wherein the first antibody specifically binds to glycosyl transferase d, heparin-binding hemagglutinin adhesin, methylated heparin-binding hemagglutinin adhesin or ferric reductase.

6. A method according to any one of the preceding claims herein the first antibody specifically binds a protein of M. avium subspecies paratuberculosis or M. tuberculosis, wherein the protein of M. avium subspecies paratuberculosis is optionally glycosyl transferase d or heparin-binding hemagglutinin adhesion and the protein of M. tuberculosis is optionally methylated heparin-binding hemagglutinin adhesin or ferric reductase.

7. A method according to any one of the preceding claims wherein the first and/or second antibodies are monoclonal antibodies, or are in the form of serum.

8. A method according to any one of the preceding claims wherein 2, 3, 4 or more different first antibodies are used, optionally labeled with different quantum dots labels.

9. A method according to claim 8 in which the different quantum dots each have a different emission spectrum.

10. A method according to any one of the preceding claims in which the sample comprises purified protein and/or a lysate and/or is from an individual who does not have any symptoms of mycobacterial infection or disease.

11. A method according to any one of the preceding claims wherein the quantum dots are detected using UV light or a fluorescent plate reader.

12. A method according to any one of the preceding claims wherein the mycobacterial proteins are bound to plates, contacted with sera that contains the first antibodies, successively washed, and then binding of the first antibodies is detected by adding a biotin bounded secondary antibody IgG anti human and then contacting with streptavidin bound to a quantum dot, to allow determination of the binding of the second antibody to the first antibody.

13. A kit for carrying out the method of any one of claims 1 to 12 comprising streptavidin hound to a quantum dot and either (i) 1, 2 or 3 different first antibodies as defined in claim 1 and 1, 2 or 3 second antibodies as defined in claim 1, wherein the second antibodies are bound to biotin, or (ii) 1, 2 or 3 first antibodies as defined in claim 1 bound to biotin; wherein the kit optionally also comprises instructions for carrying out the method.


SEQUENCE LISTING

<110>  Ikonomopoulos, John

<120>  MYCOBACTERIAL PROTEIN DETECTION

<130>  epo/ikon/PDs

<160>  3

<170>  PatentIn version 3.5

<210>  1
<211>  195
<212>  PRT
<213>  Mycobacterium avium

<400>  1

Met Lys Gly Thr Thr Met Ala Glu Asn Pro Asn Ile Asp Asp Leu Arg
1               5                   10                  15

Ala Pro Leu Leu Ala Ala Leu Gly Ala Ala Asp Leu Ala Leu Ala Thr
            20                  25                  30

Val Asn Asp Leu Ile Ala Asn Leu Arg Glu Arg Ala Glu Glu Thr Arg
        35                  40                  45

Ala Glu Thr Arg Thr Arg Val Glu Glu Arg Arg Ala Arg Leu Thr Lys
    50                  55                  60

Phe Gln Glu Asp Leu Pro Glu Gln Phe Thr Glu Leu Arg Asp Lys Phe
65                  70                  75                  80

Thr Thr Glu Glu Leu Arg Lys Ala Ala Glu Gly Tyr Leu Glu Ala Ala
                85                  90                  95

Thr Asn Arg Tyr Asn Glu Leu Val Glu Arg Gly Glu Ala Ala Leu Gln
            100                 105                 110

Arg Leu Arg Ser Gln Thr Ala Phe Glu Asp Ala Ser Ala Arg Ala Glu
        115                 120                 125

Gly Tyr Val Asp Gln Ala Val Glu Leu Thr Gln Glu Ala Leu Gly Thr
    130                 135                 140

Val Ala Ser Gln Thr Arg Ala Val Gly Glu Arg Ala Ala Lys Leu Val
145                 150                 155                 160

Gly Ile Glu Leu Pro Gly Lys Ala Glu Ala Ala Gly Lys Lys Ala Gln

165                 170                 175

Lys Ala Ile Ala Lys Ala Pro Ala Lys Lys Ala Pro Ala Lys Lys Val
            180                 185                 190

Thr Gln Lys
        195

<210>  2
<211>  266
<212>  PRT
<213>  Mycobacterium avium

<400>  2

Met Thr Ala Pro Val Phe Ser Ile Ile Ile Pro Thr Phe Asn Ala Ala
1               5                   10                  15

Val Thr Leu Gln Ala Cys Leu Gly Ser Ile Val Gly Gln Thr Tyr Arg
            20                  25                  30

Glu Val Glu Val Val Leu Val Asp Gly Gly Ser Thr Asp Arg Thr Leu
        35                  40                  45

Asp Ile Ala Asn Ser Phe Arg Pro Glu Leu Gly Ser Arg Leu Val Val
    50                  55                  60

His Ser Gly Pro Asp Asp Gly Pro Tyr Asp Ala Met Asn Arg Gly Val
65                  70                  75                  80

Gly Val Ala Thr Gly Glu Trp Val Leu Phe Leu Gly Ala Asp Asp Thr
                85                  90                  95

Leu Tyr Glu Pro Thr Thr Leu Ala Gln Val Ala Ala Phe Leu Gly Asp
            100                 105                 110

His Ala Ala Ser His Leu Val Tyr Gly Asp Val Val Met Arg Ser Thr
        115                 120                 125

Lys Ser Arg His Ala Gly Pro Phe Asp Leu Asp Arg Leu Leu Phe Glu
    130                 135                 140

Thr Asn Leu Cys His Gln Ser Ile Phe Tyr Arg Arg Glu Leu Phe Asp
145                 150                 155                 160

Gly Ile Gly Pro Tyr Asn Leu Arg Tyr Arg Val Trp Ala Asp Trp Asp
                165                 170                 175

Phe Asn Ile Arg Cys Phe Ser Asn Pro Ala Leu Ile Thr Arg Tyr Met
            180                 185                 190

Asp Val Val Ile Ser Glu Tyr Asn Asp Met Thr Gly Phe Ser Met Arg
        195                 200                 205

Gln Gly Thr Asp Lys Glu Phe Arg Lys Arg Leu Pro Met Tyr Phe Trp
    210                 215                 220

Val Ala Gly Trp Glu Thr Cys Arg Arg Met Leu Ala Phe Leu Lys Asp
225                 230                 235                 240

Lys Glu Asn Arg Arg Leu Ala Leu Arg Thr Arg Leu Ile Arg Val Lys
                245                 250                 255

Ala Val Ser Lys Glu Arg Ser Ala Glu Pro
            260                 265

<210>  3
<211>  456
<212>  PRT
<213>  Mycobacterium tuberculosis

<400>  3

Met Arg Pro Tyr Tyr Ile Ala Ile Val Gly Ser Gly Pro Ser Ala Phe
1               5                   10                  15

Phe Ala Ala Ala Ser Leu Leu Lys Ala Ala Asp Thr Thr Glu Asp Leu
            20                  25                  30

Asp Met Ala Val Asp Met Leu Glu Met Leu Pro Thr Pro Trp Gly Leu
        35                  40                  45

Val Arg Ser Gly Val Ala Pro Asp His Pro Lys Ile Lys Ser Ile Ser
    50                  55                  60

Lys Gln Phe Glu Lys Thr Ala Glu Asp Pro Arg Phe Arg Phe Phe Gly
65                  70                  75                  80

Asn Val Val Val Gly Glu His Val Gln Pro Gly Glu Leu Ser Glu Arg
                85                  90                  95

Tyr Asp Ala Val Ile Tyr Ala Val Gly Ala Gln Ser Asp Arg Met Leu
            100                 105                 110

Asn Ile Pro Gly Glu Asp Leu Pro Gly Ser Ile Ala Ala Val Asp Phe
        115                 120                 125

Val Gly Trp Tyr Asn Ala His Pro His Phe Glu Gln Val Ser Pro Asp
    130                 135                 140

Leu Ser Gly Ala Arg Ala Val Val Ile Gly Asn Gly Asn Val Ala Leu
145                 150                 155                 160

Asp Val Ala Arg Ile Leu Leu Thr Asp Pro Asp Val Leu Ala Arg Thr
                165                 170                 175

Asp Ile Ala Asp His Ala Leu Glu Ser Leu Arg Pro Arg Gly Ile Gln
            180                 185                 190

Glu Val Val Ile Val Gly Arg Arg Gly Pro Leu Gln Ala Ala Phe Thr
        195                 200                 205

Thr Leu Glu Leu Arg Glu Leu Ala Asp Leu Asp Gly Val Asp Val Val
    210                 215                 220

Ile Asp Pro Ala Glu Leu Asp Gly Ile Thr Asp Glu Asp Ala Ala Ala
225                 230                 235                 240

Val Gly Lys Val Cys Lys Gln Asn Ile Lys Val Leu Arg Gly Tyr Ala
                245                 250                 255

Asp Arg Glu Pro Arg Pro Gly His Arg Arg Met Val Phe Arg Phe Leu
            260                 265                 270

Thr Ser Pro Ile Glu Ile Lys Gly Lys Arg Lys Val Glu Arg Ile Val
        275                 280                 285

Leu Gly Arg Asn Glu Leu Val Ser Asp Gly Ser Gly Arg Val Ala Ala
    290                 295                 300

Lys Asp Thr Gly Glu Arg Glu Glu Leu Pro Ala Gln Leu Val Val Arg
305                 310                 315                 320

Ser Val Gly Tyr Arg Gly Val Pro Thr Pro Gly Leu Pro Phe Asp Asp
                325                 330                 335

Gln Ser Gly Thr Ile Pro Asn Val Gly Gly Arg Ile Asn Gly Ser Pro
            340                 345                 350

Asn Glu Tyr Val Val Gly Trp Ile Lys Arg Gly Pro Thr Gly Val Ile
        355                 360                 365

Gly Thr Asn Lys Lys Asp Ala Gln Asp Thr Val Asp Thr Leu Ile Lys
    370                 375                 380

Asn Leu Gly Asn Ala Lys Glu Gly Ala Glu Cys Lys Ser Phe Pro Glu
385                 390                 395                 400

Asp His Ala Asp Gln Val Ala Asp Trp Leu Ala Ala Arg Gln Pro Lys
                405                 410                 415

Leu Val Thr Ser Ala His Trp Gln Val Ile Asp Ala Phe Glu Arg Ala
            420                 425                 430

Ala Gly Glu Pro His Gly Arg Pro Arg Val Lys Leu Ala Ser Leu Ala
        435                 440                 445

Glu Leu Leu Arg Ile Gly Leu Gly
    450                 455